# EUROPEAN PATENT APPLICATION

(11) **EP 2 777 519 A1**
(43) Date of publication of application: **17.09.2014**
(21) Application number: 14159728.6
(22) Date of filing: 14.03.2014
(51) Int. Cl.: A61B 17/068, A61B 17/115

(54) **Surgical stapling device with detachable handle sections**

(30) Priority: 15.03.2013 US 201361790921 P; 05.03.2014 US 201414197751
(71) Applicant: Covidien LP, Mansfield, MA 02048 (US)
(72) Inventor: Tanner, Mark, London SE3 9HX (GB); Wilson, Robbie, Harwich, Essex CO12 5NW (GB)
(74) Representative: Soames, Candida Jane

(57) **Abstract**

A surgical stapling device (10) including a frame (30) configured to support a proximal portion of an approximation assembly (100) and a proximal portion of a staple pusher assembly (200) and a pair of handle sections (20a,20b) pivotally secured to the frame (30) such that the handle sections can be moved from a closed position wherein the handle sections enclose the proximal portion of the approximation assembly and the staple pusher assembly and an open position wherein the proximal portion of the approximation assembly and the staple pusher assembly are exposed.

## Description

### CROSS-REFERENCE TO RELATED APPLICATIONS

This application claims the benefit of and priority to U.S. Provisional Patent Application No. 61/790,921, filed March 15, 2013, the entire disclosure of which is incorporated by reference herein.

### Background

### 1. Technical Field

The present disclosure relates to a surgical fastener applying apparatus and, more particularly, to surgical fastener applying apparatus having reusable and disposable components.

### 2. Discussion of Related Art

Anastomosis refers to the surgical joining of separate hollow tissue sections. Typically, an anastomosis procedure follows surgery in which a diseased or defective section of a hollow tissue structure is removed, thus requiring the joining of the remaining end sections of the tissue structure. Depending on the particular procedure being performed and/or other factors, the end sections of the tissue may be joined by circular anastomosis, e.g., end-to-end anastomosis, end-to-side anastomosis, or side-to-side anastomosis.

In a circular anastomosis procedure, the two end sections of a tubular organ are joined using a stapling apparatus that drives a circular array of staples through each of the end sections to join the end sections to one another in end-to-end relation and simultaneously cores any tissue within the newly joined hollow tissue structure to clear the passage defined by the hollow tissue structure. The apparatus can also apply other surgical fasteners such as, for example, clips or two part polymeric surgical fasteners.

A typical circular anastomosis apparatus includes an elongated shaft having a handle portion at a proximal end and a staple holding component at a distal end. An anvil assembly including an anvil rod and an attached anvil head is mounted to the distal end of the elongated shaft adjacent the staple holding component. In use, the end portions to be joined are clamped between the anvil head and the staple holding component. The clamped end portions are then joined to one another by driving one or more staples from the staple holding component, through the tissue, and into the anvil head to form the staples about the tissue. In some such apparatus, a knife is provided to cut the tissue which has been joined by the staples. An example of such a circular anastomosis apparatus is described in U.S. Patent No. 7,857,187 and 7,303,106 to Milliman, the entire contents of which are hereby incorporated by reference herein in their entirety.

Because of the risks associated with improper sterilization, surgical fastener apparatus are typically disposable after use. Although the cartridge assembly may be replaced to perform multiple fastener applying operations on a single patient, the staple applying apparatus is typically disposable after a surgical procedure has been completed. This requirement of disposability may increase the costs associated with surgical procedures. Although reusable fastener applying apparatus have been developed, such apparatus can be overly complex and prove difficult to sterilize. A need exists for a surgical stapling apparatus that includes reusable components and is not overly complex and is configured to facilitate sterilization after use in a surgical procedure.

### Summary

Accordingly, a reusable surgical stapling device is provided. The stapling device includes a frame configured to support a proximal portion of an approximation assembly and a proximal portion of a staple pusher assembly and a pair of handle sections pivotally secured to the frame such that the handle sections can be moved from a closed position wherein the handle sections enclose the proximal portion of the approximation assembly and the staple pusher assembly and an open position wherein the proximal portion of the approximation assembly and the staple pusher assembly are exposed. The approximation assembly is configured to approximate an anvil assembly relative to a staple assembly and includes a selectively removable rotatable knob. The staple pusher assembly includes a pusher linkage for effecting ejection of a plurality of staples from a staple assembly.

In some embodiments, the frame includes an attachment portion configured for selective engagement with the pair of handle sections. A proximal extension of the frame may include a pair of annular flanges configured to be received about a rotatable sleeve of the approximation assembly. A distal extension of the frame may include an annular flange configured to be received about an elongated pusher tube of the staple pusher assembly.

In other embodiments, the frame may include a trap detail for stabilizing a firing trigger of the staple pusher assembly when the housing is in the open position. The trap detail may include a pair of legs extending from the base in alignment with a firing link of the firing trigger of the staple pusher assembly. In one embodiment, each leg of the trap detail is a mirror image of the other. Each leg may define an elongated slot configured to receive a pivot member. The pivot member may pivotally connect the firing link of the firing trigger with the handle sections of the housing. Each of the legs may operate as a stop feature. In one embodiment, a distal facing surface of each of the legs is configured to engage a tab of a screw stop of the approximation assembly when the screw stop has reached a fully retracted position.

Also disclosed is a method of disassembling a surgical stapler. The method includes a step of providing a surgical stapler including a housing, an approximation assembly, a staple pusher assembly, an outer tube, and a ferrule securing the outer tube to the housing, wherein the housing includes handle sections pivotally secured to a frame. The method further includes the steps of disengaging the ferrule from the handle sections of the housing, opening the housing by pivoting the handle sections relative to the frame, separating the outer tube from the housing, and disengaging the approximation assembly and the staple pusher assembly from the frame.

### Brief Description of the Drawings

Various embodiments of the presently disclosed surgical fastener applying apparatus will now be described herein with reference to the accompanying figures wherein:
FIG. 1 is a perspective view of an embodiment of the presently disclosed surgical stapling device;
FIG. 2 is a perspective side view of the surgical stapling device shown in FIG. 1, with the housing, outer tube, ferrule and stapling assembly removed;
FIG. 2A is an enlarged portion of the indicated area of detail shown in FIG. 2;
FIG. 3 is an enlarged side perspective view of the proximal portion of the stapling device as shown in FIG. 2;
FIG. 4 is an enlarged perspective end view of the proximal portion of the stapling device as shown in FIG. 3;
FIG. 5 is an enlarged perspective end view of the proximal portion of the stapling device as shown in FIG. 3, with handle sections attached;
FIG. 6 is a perspective view of the surgical stapling device of FIG. 1 with the staple assembly removed;
FIG. 7 is a perspective view of the surgical stapling device of FIG. 6 with the ferrule disengaged from the housing;
FIG. 8 is a perspective view of the surgical stapling device of FIG. 7 with the handle sections opened; and
FIG. 9 is a perspective view of the surgical stapling device of FIG. 8 with the outer tube and ferrule removed.

### Detailed Description of Embodiments

Embodiments of the presently disclosed surgical stapling device will now be described in detail with reference to the drawings in which like reference numerals designate identical or corresponding elements in each of the several views. Throughout this description, the term "proximal" will refer to the portion of the instrument closer to the operator and the term "distal" will refer to the portion of the instrument further from the operator.

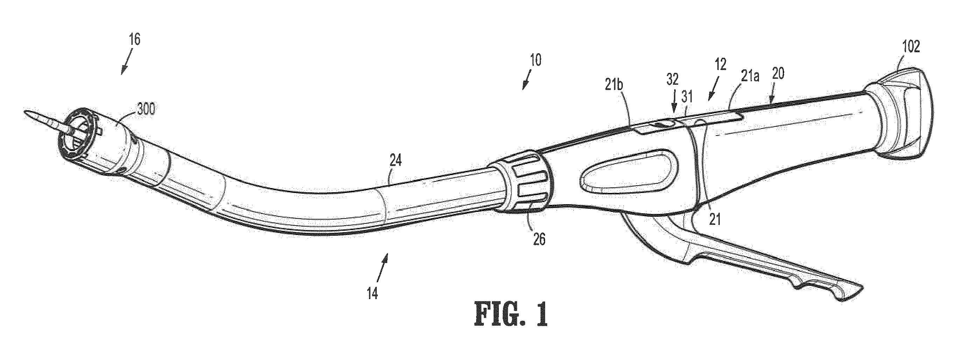

FIGS. 1-9 illustrate one embodiment of the presently disclosed surgical fastener applying apparatus designated generally as surgical stapling device 10. Stapling device 10 is intended to, at least in part, be resterilized and reused. As will become apparent from the following disclosure, the construction of stapling device 10 facilitates the sterilization of internal components of stapling device 10 between uses.

With reference initially to FIG. 1, stapling device 10 includes a proximal handle portion 12, an elongated curved central body portion 14 and a distal head portion 16. Alternately, in some surgical procedures, e.g., the treatment of hemorrhoids, it may be desirable to have a substantially straight, shortened, central body portion. It is envisioned that the length, shape and/or the diameter of any of handle portion 12, body portion 14 and head portion 16 may also be varied to suit a particular surgical procedure. It is also envisioned that the handle portion may be replaced with an adapter for securing stapling device 10 to a robotic arm to facilitate robotic operation of stapling device 10.

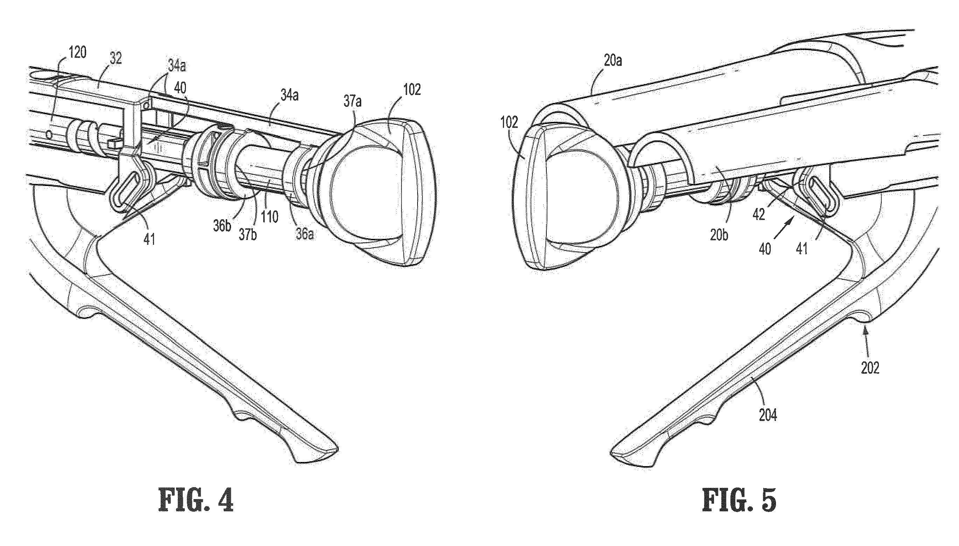

With reference now to FIGS. 1-3, handle portion 12 of stapling device 10 includes a housing 20 which supports proximal portions of an approximation assembly 100 and a staple pusher assembly 200. Approximation assembly 100 and staple assembly 200 will only be described to the extent necessary to fully disclose the aspects of the present disclosure. For a more detailed discussion of approximation assembly 100 and staple pushing assembly 200 please refer to commonly owned U.S. Patent Nos. 7,857,187 to Milliman ("the Milliman '187 patent" and 6,945,444 to Gresham et al. ("the Gresham '444 patent"), the contents of each of which is incorporated herein by reference in its entirety. As will be discussed in further detail below, housing 20 includes a pair of handle sections 20a, 20b (FIG. 5) which are configured to pivot about a longitudinal axis to permit opening of housing 20. As will also be discussed in further detail below, removal of handle sections 20a, 20b facilitates sterilization of stapling device 10.

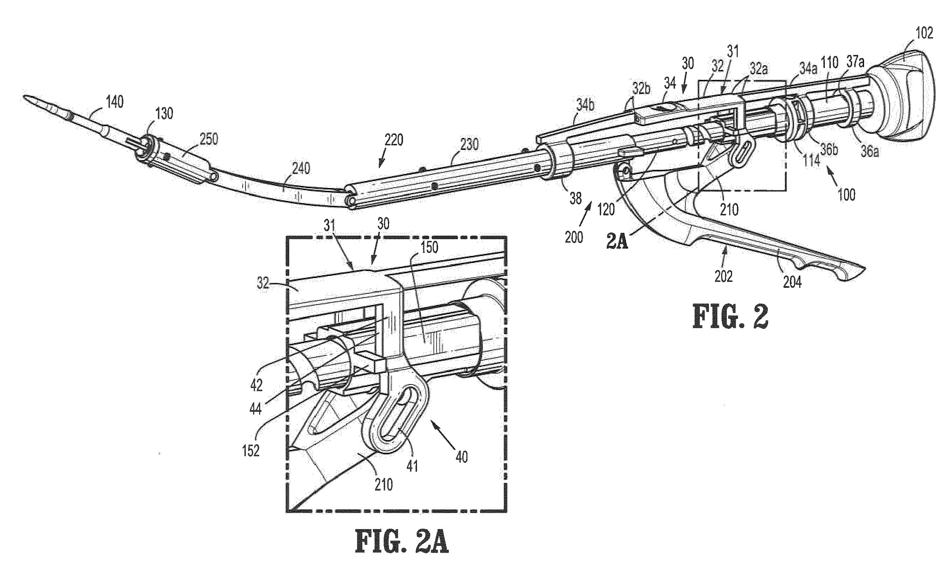

With reference to FIGS. 1 and 2, body portion 14 of stapling device 10 includes an outer tube 24 which supports elongated portions of approximation assembly 100 and staple pusher assembly 200, and a ferrule 26 configured to selectively secure handle sections 20a, 20b of housing 20 together and to secure outer tube 24 to housing 20. Head portion 16 of stapling device 10 includes a staple assembly 300 which is releasably supported on a distal end of outer tube 24 and receives a distal end of staple pusher assembly 200. To allow for repeated use of surgical stapling device 10, each component of surgical stapling device 10 is configured to be sterilized or to be replaced. In one embodiment, each of housing 20, outer tube 24, ferrule 26, approximation assembly 100 and staple pusher assembly 200 are configured to be reused while staple assembly 300 is configured to be replaced. As such, except where otherwise noted, the components of surgical stapling device 10 are generally formed from thermoplastics including polycarbonates, and metals including stainless steel and aluminum, that are suited to withstand repeated sterilization procedures, e.g., autoclaving.

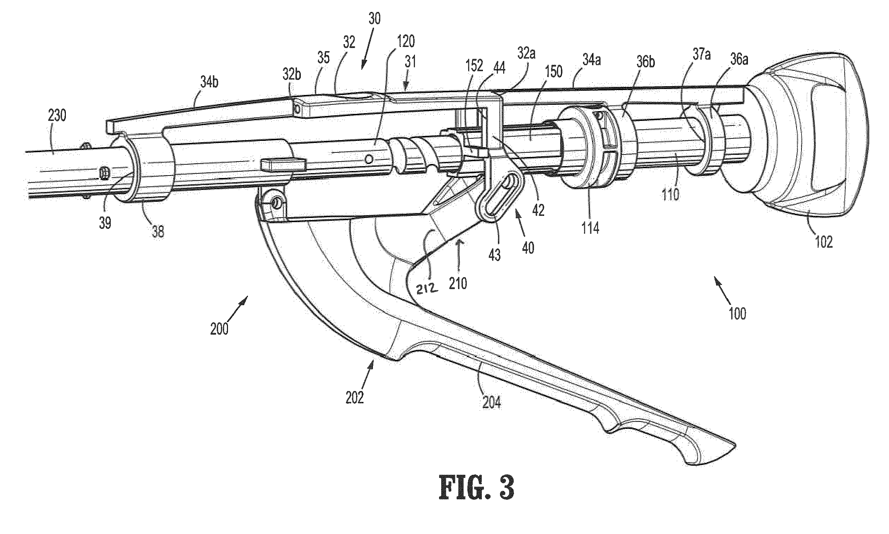

With reference now to FIGS. 2 and 3, stapling device 10 is shown with handle sections 20a, 20b of housing 20 and outer tube 24 removed to expose approximation assembly 100 and staple pusher assembly 200. Briefly, approximation assembly 100 includes a rotatable knob 102 which is configured for operable engagement by a user, a rotatable sleeve 110 extending distally from rotatable knob 102, a substantially cylindrical collar 114 mounted on a distal end of rotatable sleeve 110, a screw 120 positioned within and extending from rotatable sleeve 110, a screw extension 130 having a proximal end (not shown) operably attached to screw 120, an anvil retainer 140 operably attached to a distal end of screw extension 130, and a screw stop 150 mounted about screw 120. In one embodiment, rotatable knob 102 is integrally formed with rotatable sleeve 110. Alternatively, rotatable knob 102 may be selectively or fixedly attached to rotatable sleeve 110 with mechanical fasteners, bonding, welding, adhesives or using any other known fastening technique.

Still referring to FIGS. 2 and 3, staple pusher assembly 200 includes a firing trigger 202 and a pusher linkage 220 operably connected to firing trigger 202. Firing trigger 202 includes a lever 204 pivotally connected to pusher linkage 220 (FIG. 2) and a firing link 210 configured to be pivotally connected with housing 20 by a pivot member (not shown). As shown, firing link 210 is integrally formed with lever 204. Firing link 210 and lever 204 are pivotal relative to each other through operation of a living hinge 212 (FIG. 3). Alternatively, lever 204 and firing link 210 may be formed as separate components that are pivotally connected to each by pivot pin or by any other suitable means, such as disclosed in the Milliman '187. Pusher linkage 220 includes an elongated pusher tube 230 operably connected to firing trigger 202, a flexible tube extension 240 extending distally from elongated pusher tube 230 and an end pusher tube 250 operably connected to tube extension 240 and configured for releasable connection with staple assembly 300 (FIG. 1). Flexible tube extension 240 is positioned to move within curved outer tube 24.

With particular reference now to FIGS. 2, 2A and 3, surgical stapling device 10 includes a chassis or frame 30 operably supporting proximal portions of approximation assembly 100 and staple pusher assembly 200. Chassis 32 provides attachment for handle sections 20a, 20b of housing 20 (FIG. 1) and includes a base 31 having an attachment portion 32, a proximal extension 34a, and a distal extension 34b. Base 31 is sized to extend substantially the length of housing 20. As will be discussed in further detail below, attachment portion 32 of base 31 is configured to pivotally and releasably engage handles sections 20a, 20b of housing 20. In one embodiment, proximal and distal ends 32a, 32b of attachment portion 32 each include a recess (not shown) formed on either side of respective proximal and distal extensions 34a, 34b each configured to receive a pivot pin (not shown). Slots (not shown) formed in handle sections 20a, 20b are each configured to selectively receive the pivot pins (not shown) extending from within the recesses (not shown) formed in attachment portion 32. Alternatively, attachment portion 32 may include integrally formed tabs extending from proximal and distal ends 32a, 32b configured for receipt within the slots formed in handle sections 20a, 20b. It is envisioned that handle sections 20a, 20b may instead include the recesses to receive the pivot pins or include integrally formed tabs for receipt within the slots formed in attachment portion 32.

Proximal extension 34a includes a pair of annular flanges 36a, 36b configured to be received about rotatable sleeve 110 of approximation assembly 100. Openings 37a, 37b (FIG. 4) in each of annular flanges 36a, 36b are sized such that rotatable sleeve 110 can be rotated about its longitudinal axis during approximation and unapproximation of an anvil assembly (not shown) during use of surgical stapling device 10. Distal extension 34b of base 31 includes a single annular flange 38 (FIG. 3) configured to be received about elongated pusher tube 230 of staple pusher assembly 200. Opening 39 in annular flange 38 is sized such that elongated pusher tube 230 may be longitudinally advanced and retracted therethrough as firing trigger 202 is squeezed and released during use of surgical stapling device 10.

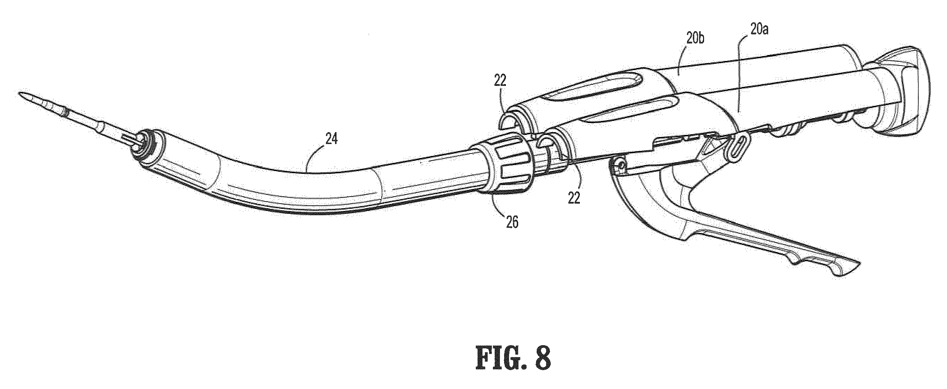

Chassis 30 further includes a trap detail 40 for stabilizing trigger 202 of staple pusher assembly 200 when housing 20 is opened. Trap detail 40 includes a pair of legs 42 extending from base 31 in alignment with firing link 210 of firing trigger 202. Each leg 42 is a mirror image of the other and defines an elongated slot 43 configured to receive the pivot member (not shown) that pivotally connects firing link 210 of firing trigger 202 with handle sections 20a, 20b of housing 20 (FIG. 1). In this manner, trap detail 40 operates to maintain firing trigger 202 in operable position once handle sections 20a, 20b of housing 20 (FIG. 8) are opened and/or removed during sterilization of surgical stapling device 10. Each of legs 42 also operates as a stop feature to prevent further proximal movement of screw stop 150 and thus, screw 120, during approximation of the anvil assembly (not shown) with staple assembly 300. In particular, a distal facing surface 44 of each of legs 42 is configured to engage a tab 152 of screw stop 150 when screw stop 150 has reached a fully retracted position.

Turning now to FIGS. 1, 2, 7 and 8, handle sections 20a, 20b of housing 20 are pivotally connected to attachment portion 32 of base 31. Handle sections 20a, 20b may be operably attached to chassis 30 with mechanical fasteners, snap-fit engagement, or any other suitable method. As discussed above, and as shown, each of handle sections 20a, 20b defines a notch 21 (FIG. 1). Proximal and distal ends 21a, 21b of notch 21 each define a slot (not shown) configured to selectively engage a pivot pin (not shown) extending from within each of the recesses (not shown) formed in distal ends 32a, 32b of attachment portion 32 of base 31. Once the pivot pins extending from attachment portion 32 are received within the corresponding slots formed in handle sections 20a, 20b, handle sections 20a, 20b may be pivoted back and forth between an open position (FIG. 8) and a closed position (FIG. 1).

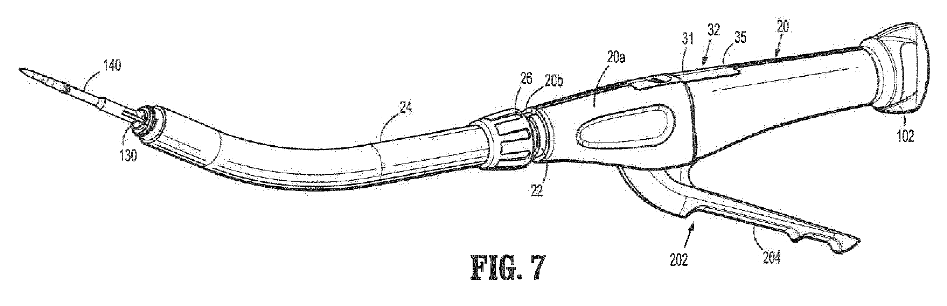

With particular reference to FIGS. 7 and 8, distal ends of handle sections 20a, 20b include an annular thread 22 configured to be selectively engaged by ferrule 26. Engagement of annular thread 22 by ferrule 26 secures handle sections 20a, 20b together in the closed position (FIG. 1). It is envisioned that handle sections 20a, 20b may further include one or more snap-fit fasteners or other mechanisms for selectively securing handle sections 20a, 20b to one another.

Although not shown, each of handle sections 20a, 20b define one or more channels and one or more recesses on an inner surface thereof configured to engage various components of approximation assembly 100 and staple pusher assembly 200 when handle sections 20a, 20b are in the closed position (FIG. 1).

The disassembly of surgical stapling device 10 will now be described with reference to FIGS. 1, 2 and 6-9. Referring initially to FIG. 1, stapling device 10 is shown in a pre-fired or post-fired condition. For purposes of the following discussion, stapling device 10 will be considered to be in a post-fired condition. Handle section 20a, 20b (FIG. 8) of housing 20 are secured together by ferrule 26, outer tube 24 is secured to housing 20 by ferrule 26 and staple assembly 300 is secured to outer tube 24 using for example, screw threads as will be discussed in further detail below. Subsequent to use, an anvil assembly (not shown) is unapproximated in relation to staple assembly 300 using approximation assembly 100 and removed from anvil retainer 140.

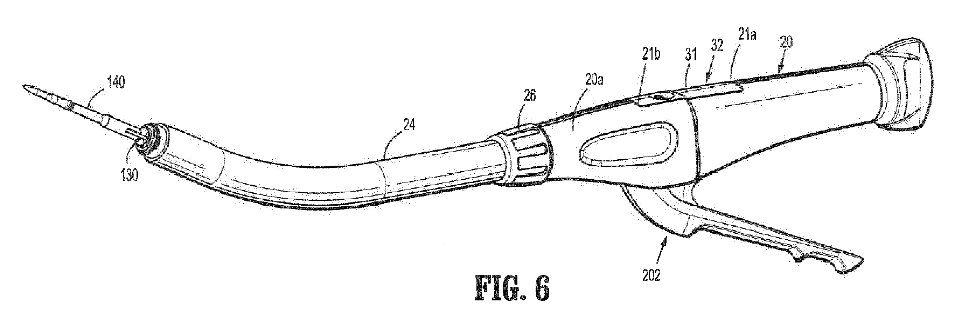

Turning to FIG. 6, staple assembly 300 and outer tube 24 may be threaded together, snap-fit, bayonet coupled or releasably secured together in any suitable manner. It is envisioned that the aspects of the present disclosure may be modified for use with alternative staple assemblies and engagement configurations.

With reference now to FIG. 7, ferrule 26 is unscrewed from annular thread 22 to disengage ferrule 26 from handle sections 20a, 20b and to disengage outer tube 24 from housing 20 to release handle sections 20a, 20b. Although shown and described as being a threaded connection, it is envisioned that ferrule 26 and handle sections 20a, 20b may instead be joined using a bayonet coupling, friction fit or by using another fastening technique.

Handle sections 20a, 20b are next opened as shown in FIG. 8 to release outer tube 24 from engagement with housing 20. As noted above, handle sections 20a, 20b may include one or more additional fastening means for securing handle sections 20a, 20b together which may require releasing prior to opening of housing 20. Opening of housing 20 exposes the proximal portions of approximation assembly 100 and staple pusher assembly 200 (FIG. 2) for sterilization.

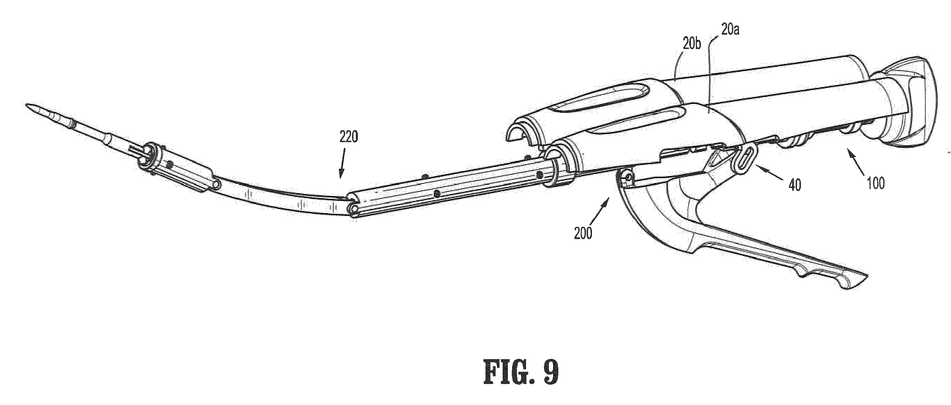

Once housing sections 20a, 20b are opened, outer tube 24 is disengaged from housing 20 and removed (see FIG. 9). Removal of outer tube 24 exposes elongated portions of approximation assembly 100 and staple pusher assembly 200 (FIG. 2).

With reference again to FIG. 2, handle sections 20a, 20b (FIG. 1) may then be separated from attachment portion 32 of chassis 30 to facilitate sterilization of handle sections 20a, 20b, chassis 30, approximation assembly 100 and staple pusher assembly 200. As noted above, in some embodiments, rotatable knob 102 may also be removed to facilitate sterilization. Once sterilized, surgical stapling device 10 may be reassembled in the reverse manner of disassembly.

It is also contemplated that in alternate embodiments, the handle sections 20a, 20b are opened but remain attached to the chassis during disassembly and sterilization.

It is noted that because staple assembly 300 can be easily separated from outer tube 24 and approximation assembly 100, and replaced with a fresh staple assembly 300, stapling device 10 may be used to perform multiple operations during a single surgical procedure on a single patient without resterilizing stapling device 10.

It will be understood that various modifications may be made to the embodiments disclosed herein. Therefore, the above description should not be construed as limiting, but merely as exemplifications of preferred embodiments. Those skilled in the art will envision other modifications within the scope and spirit of the claims appended hereto. For example, although the description refers exclusively to staples, it is envisioned that staples may include different types of tissue fasteners including two-part fasteners. In a stapling device for applying two-part fastener, the anvil assembly of the stapling device would support one part of each two-part fastener.

The invention may be described by reference to the following numbered paragraphs: -
1. A surgical stapling device comprising:
   a frame configured to support a proximal portion of an approximation assembly and a proximal portion of a staple pusher assembly;
   a pair of handle sections pivotally secured to the frame such that the handle sections can be moved from a closed position wherein the handle sections enclose the proximal portion of the approximation assembly and the staple pusher assembly and an open position wherein the proximal portion of the approximation assembly and the staple pusher assembly are exposed.
2. The surgical stapling device of paragraph 1, wherein the approximation assembly is configured to approximate an anvil assembly relative to a staple assembly.
3. The surgical stapling device of paragraph 2, wherein the approximation assembly includes a selectively removable rotatable knob.
4. The surgical stapling device of paragraph 1, wherein the staple pusher assembly includes a pusher linkage for effecting ejection of a plurality of staples from a staple assembly.
5. The surgical stapling device of paragraph 1, wherein the frame includes an attachment portion configured for selective engagement with the pair of handle sections.
6. The surgical stapling device of paragraph 1, wherein a proximal extension of the frame includes a pair of annular flanges configured to be received about a rotatable sleeve of the approximation assembly.
7. The surgical stapling device of paragraph 1, wherein a distal extension of the frame includes an annular flange configured to be received about an elongated pusher tube of the staple pusher assembly.
8. The surgical stapling device of paragraph 1, wherein the frame includes a trap detail for stabilizing a firing trigger of the staple pusher assembly when the housing is in the open position.
9. The surgical stapling device of paragraph 8, wherein the trap detail includes a pair of legs extending from the base in alignment with a firing link of the firing trigger of the staple pusher assembly.
10. The surgical stapling device of paragraph 9, wherein each leg is a mirror image of the other.
11. The surgical stapling device of paragraph 9, wherein each leg defines an elongated slot configured to receive a pivot member.
12. The surgical stapling device of paragraph 11, wherein the pivot member pivotally connects the firing link of the firing trigger with the handle sections of the housing.
13. The surgical stapling device of paragraph 9, wherein each of the legs operates as a stop feature.
14. The surgical stapling device of paragraph 13, wherein a distal facing surface of each of the legs is configured to engage a tab of a screw stop of the approximation assembly when the screw stop has reached a fully retracted position.
15. A surgical stapling device comprising:
   an approximation assembly;
      a pusher assembly;
      a chassis removably attached to the approximation assembly; and
   a pair of handle sections pivotally secured to the chassis such that the handle sections can be moved from a closed position wherein the handle sections enclose a portion of the approximation assembly and an open position wherein the portion of the approximation assembly is exposed.
16. The surgical stapling device of paragraph 15 wherein the chassis is removably attached to the pusher assembly, and in the closed position the handle sections enclose a portion of the stapler pusher assembly.
17. The surgical stapling device of paragraph 15, wherein the chassis includes a first clamping member to removably clamp a sleeve of an approximation assembly.
18. The surgical stapling device of paragraphs 16, wherein the chassis includes a second clamping member to removably clamp a pusher tube of the pusher assembly.
19. A method of disassembling a surgical stapler comprising the steps of:
   providing a surgical stapler including a housing, an approximation assembly, a staple pusher assembly, an outer tube, and a ferrule securing the outer tube to the housing, wherein the housing includes handle sections pivotally secured to a frame;
   disengaging the ferrule from the handle sections of the housing;
   opening the housing by pivoting the handle sections relative to the frame;
   separating the outer tube from the housing; and
   disengaging the approximation assembly and the staple pusher assembly from the frame.

## Claims

1. A surgical stapling device comprising:
a frame configured to support a proximal portion of an approximation assembly and a proximal portion of a staple pusher assembly;
a pair of handle sections pivotally secured to the frame such that the handle sections can be moved from a closed position wherein the handle sections enclose the proximal portion of the approximation assembly and the staple pusher assembly and an open position wherein the proximal portion of the approximation assembly and the staple pusher assembly are exposed.

2. The surgical stapling device of claim 1, wherein the approximation assembly is configured to approximate an anvil assembly relative to a staple assembly, wherein the approximation assembly includes a selectively removable rotatable knob.

3. The surgical stapling device of claim 1 or claim 2, wherein the staple pusher assembly includes a pusher linkage for effecting ejection of a plurality of staples from a staple assembly.

4. The surgical stapling device of any preceding claim, wherein the frame includes an attachment portion configured for selective engagement with the pair of handle sections.

5. The surgical stapling device of any preceding claim, wherein a proximal extension of the frame includes a pair of annular flanges configured to be received about a rotatable sleeve of the approximation assembly.

6. The surgical stapling device of any preceding claim, wherein a distal extension of the frame includes an annular flange configured to be received about an elongated pusher tube of the staple pusher assembly.

7. The surgical stapling device of any preceding claim, wherein the frame includes a trap detail for stabilizing a firing trigger of the staple pusher assembly when the housing is in the open position, preferably wherein the trap detail includes a pair of legs extending from the base in alignment with a firing link of the firing trigger of the staple pusher assembly.

8. The surgical stapling device of claim 7, wherein each leg is a mirror image of the other, preferably wherein each leg defines an elongated slot configured to receive a pivot member, preferably still wherein the pivot member pivotally connects the firing link of the firing trigger with the handle sections of the housing.

9. The surgical stapling device of claim 8, wherein each of the legs operates as a stop feature, preferably wherein a distal facing surface of each of the legs is configured to engage a tab of a screw stop of the approximation assembly when the screw stop has reached a fully retracted position.

10. A surgical stapling device comprising:
an approximation assembly;
a pusher assembly;
a chassis removably attached to the approximation assembly; and
a pair of handle sections pivotally secured to the chassis such that the handle sections can be moved from a closed position wherein the handle sections enclose a portion of the approximation assembly and an open position wherein the portion of the approximation assembly is exposed.

11. The surgical stapling device of claim 10 wherein the chassis is removably attached to the pusher assembly, and in the closed position the handle sections enclose a portion of the stapler pusher assembly, preferably wherein the chassis includes a first clamping member to removably clamp a sleeve of an approximation assembly.

12. The surgical stapling device of claims 11, wherein the chassis includes a second clamping member to removably clamp a pusher tube of the pusher assembly.

13. A method of disassembling a surgical stapler comprising the steps of:
providing a surgical stapler including a housing, an approximation assembly, a staple pusher assembly, an outer tube, and a ferrule securing the outer tube to the housing, wherein the housing includes handle sections pivotally secured to a frame;
disengaging the ferrule from the handle sections of the housing;
opening the housing by pivoting the handle sections relative to the frame;
separating the outer tube from the housing; and
disengaging the approximation assembly and the staple pusher assembly from the frame.
